# EUROPEAN PATENT SPECIFICATION

(11) **EP 1 543 026 B1**
(45) Date of publication and mention of the grant of the patent: **02.08.2006**
(21) Application number: 03750606.0
(22) Date of filing: 23.09.2003
(51) Int. Cl.: C07K 14/21, C12P 19/42, C12N 15/31, C12N 5/10

(54) **TRANSCRIPTIONAL ACTIVATOR GENE FOR GENES INVOLVED IN COBALAMIN BIOSYNTHESIS**
TRANSKRIPTIONSAKTIVATOR-GEN FÜR GENE, DIE BETEILIGT SIND AN DER BIOSYNTHESE VON COBALAMIN
GENE D'ACTIVATEUR DE TRANSCRIPTION DESTINE AUX GENES IMPLIQUES DANS LA BIOSYNTHESE DE LA COBALAMINE

(30) Priority: 27.09.2002 EP 02021603
(43) Date of publication of application: 22.06.2005
(73) Proprietor: DSM IP Assets B.V., 6411 TE Heerlen (NL)
(72) Inventor: HOSHINO, Tatsuo, Kanagawa-ken, Kanagawa 248-0027 (JP); SETOGUCHI, Yutaka, Kanagawa-ken, Kanagawa 251-0033 (JP); TOMIYAMA, Noribumi, Kanagawa-ken, Kanagawa 251-0015 (JP)
(74) Representative: Keller, Günter
(86) International application number: PCT/EP2003/010572
(87) International publication number: WO 2004/031227

(56) References cited:
- WO-A-94/13813
- FR-A- 2 780 733
- DATABASE EMBL [Online] 294800 bp DNA linear, 5 July 2001 (2001-07-05) CAPELA D ET AL.: "Sinorhizobium meliloti 1021 complete chromosome; segment 8/12" Database accession no. AL591789 XP002271918 cited in the application -& CAPELA D. ET AL.: "Analysis of the chromosome sequence of the legume symbiont Sinorhizobium meliloti strain 1021" PROCEEDINGS OF THE NATIONAL ACADEMY OF SCIENCES OF THE UNITED STATES, vol. 98, no. 17, 14 August 2001 (2001-08-14), pages 9877-9882, XP002271919
- RAUX EVELYNE ET AL: "Salmonella typhimurium cobalamin (vitamin B-12) biosynthetic genes: Functional studies in S. typhimurium and Escherichia coli" JOURNAL OF BACTERIOLOGY, vol. 178, no. 3, 1996, pages 753-767, XP002271915 ISSN: 0021-9193
- BOBIK T A ET AL: "A SINGLE REGULATORY GENE INTEGRATES CONTROL OF VITAMIN B12 SYNTHESIS AND PROPANEDIOL DEGRADATION" JOURNAL OF BACTERIOLOGY, US, vol. 174, no. 7, 1 April 1992 (1992-04-01), pages 2253-2266, XP000579777 ISSN: 0021-9193
- CHEN PING ET AL: "The End of the cob Operon: Evidence that the Last Gene (cobT) Catalyzes Synthesis of the Lower Ligand of Vitamin B-12, Dimethylbenzimidazole" JOURNAL OF BACTERIOLOGY, vol. 177, no. 6, 1995, pages 1461-1469, XP002271916 ISSN: 0021-9193
- RODIONOV DMITRY A ET AL: "Comparative genomics of the vitamin B12 metabolism and regulation in prokaryotes." THE JOURNAL OF BIOLOGICAL CHEMISTRY. US, vol. 278, no. 42, 17 October 2003 (2003-10-17), pages 41148-41159, XP002271917 ISSN: 0021-9258

### SEQUENCE LISTING

<110> DSM IP ASSETS B.V.
<120> Transcriptional activator gene for genes involved in cobalamin biosynthesis
<130> NDR5233
<140> PCT/EP03/10572
   <141> 2003-09-23
<150> EP 02021603.2
   <151> 2002-09-27
<160> 8
<170> PatentIn version 3.2
<210> 1
   <211> 1439
   <212> DNA
   <213> Pseudomonas denitrificans
<220>
   <221> CDS
   <222> (303)..(1304)
<400> 1
   cttttaccga cacccgcacc agccgtgtga agcgtcgcaa tcggcaaaca caaccctgga      60
   gagcgctttg cgaatgttgc gtatctctgg cagttcttgt ccgatagcgt caacagcgct     120
   gtaaaaactg tcgctttcct tacaaaagcc tgaaagcggg gccgagccgc ctttcaccgc     180
   gcgatgtcgc aatgcgaaat ctctttgcgg tcgctgctat ccatgcgaat gtcgcaaaca     240
   gacaagccga agagtatctt ccaagcgacg aatataccgc atcgccaagc atgggaagcc     300
   tc atg aac aag cca ctg acc aaa aag cgt tct ctc gtc ttc ttc ctg       347
      Met Asn Lys Pro Leu Thr Lys Lys Arg Ser Leu Val Phe Phe Leu
      1               5                   10                  15
   gtg ccg aac ttt tcc atg ctg ccc ttt tcg gcg gcg atc gaa acg ctc      395
   Val Pro Asn Phe Ser Met Leu Pro Phe Ser Ala Ala Ile Glu Thr Leu
                   20                  25                  30
   cgc atc gcc aac cgc atg ctc ggc tac gag gcc tat tcc tgg cgc ctc      443
   Arg Ile Ala Asn Arg Met Leu Gly Tyr Glu Ala Tyr Ser Trp Arg Leu
               35                  40                  45
   gca tcg tcc gac ggc gaa aag gtc ctg tcg tcg agc ggt atc gcg ctc      491
   Ala Ser Ser Asp Gly Glu Lys Val Leu Ser Ser Ser Gly Ile Ala Leu
           50                  55                  60
   gag gtc aac tcg tcg ctt gca gac gag cgc aag ttt ctc ggc ggc gaa      539
   Glu Val Asn Ser Ser Leu Ala Asp Glu Arg Lys Phe Leu Gly Gly Glu
       65                  70                  75
   aac cgc ccc tcg atg gtg ctg gtc tgt tcc ggc atc tat gtc gag gac      587
   Asn Arg Pro Ser Met Val Leu Val Cys Ser Gly Ile Tyr Val Glu Asp
   80                  85                  90                  95
   ttc aac aac aag tcg gtc aat gcc tgg ctg cgc gag gtc tac aat cgc      635
   Phe Asn Asn Lys Ser Val Asn Ala Trp Leu Arg Glu Val Tyr Asn Arg
                   100                 105                 110
   ggc gtc gcc gtc ggc agc ctc tgt acc ggc gcc cat gtg ctg gcg tcg      683
   Gly Val Ala Val Gly Ser Leu Cys Thr Gly Ala His Val Leu Ala Ser
               115                 120                 125
   gcc ggt ctt ctg acc ggc aag cgc tgc gcc atc cac tgg gaa aac ctg      731
   Ala Gly Leu Leu Thr Gly Lys Arg Cys Ala Ile His Trp Glu Asn Leu
           130                 135                 140
   ccg ggc ttt tcc gaa agc ttc ccg cag gtc gac gtc tat gcc gac ctc      779
   Pro Gly Phe Ser Glu Ser Phe Pro Gln Val Asp Val Tyr Ala Asp Leu
       145                 150                 155
   tac gaa atc gac agc aac atc tac acc tgc gcc ggc ggc acc gcc tcg      827
   Tyr Glu Ile Asp Ser Asn Ile Tyr Thr Cys Ala Gly Gly Thr Ala Ser
   160                 165                 170                 175
   ctc gac atg atg ctg aac ctg atc gac cag gat ttc ggc gag agc ctc      875
   Leu Asp Met Met Leu Asn Leu Ile Asp Gln Asp Phe Gly Glu Ser Leu
                   180                 185                 190
   gtc aac cgc gtc tgc gaa cag gcg ctg acc gat cgc gtg cgc ggg ccc      923
   Val Asn Arg Val Cys Glu Gln Ala Leu Thr Asp Arg Val Arg Gly Pro
               195                 200                 205
   cat gac cgc cag cgc ctg ccg ctg cgc gcc cgt ctc ggc gtg cag aac      971
   His Asp Arg Gln Arg Leu Pro Leu Arg Ala Arg Leu Gly Val Gln Asn
           210                 215                 220
   gcc aag gtg ctg tcc atc atc gaa ctg atg gag gca aac ctc gcc gag     1019
   Ala Lys Val Leu Ser Ile Ile Glu Leu Met Glu Ala Asn Leu Ala Glu
       225                 230                 235
   ccg ctt tcg ctg ctc gaa atc gcc gag ggc gcc gat ctc tcc cgc cgc     1067
   Pro Leu Ser Leu Leu Glu Ile Ala Glu Gly Ala Asp Leu Ser Arg Arg
   240                 245                 250                 255
   cag atc gag cgc ctc ttc cgc cag gaa atg ggc cgc tcg cct gca cgc     1115
   Gln Ile Glu Arg Leu Phe Arg Gln Glu Met Gly Arg Ser Pro Ala Arg
                   260                 265                 270
   tac tat ctc gaa atc cgc ctc gat cgc gca agg cac ctc ttg atc cag     1163
   Tyr Tyr Leu Glu Ile Arg Leu Asp Arg Ala Arg His Leu Leu Ile Gln
               275                 280                 285
   tcg tcg atg ccg gtg gtc gaa gtg gcc gta gcc tgc ggc ttc gtc tcc     1211
   Ser Ser Met Pro Val Val Glu Val Ala Val Ala Cys Gly Phe Val Ser
           290                 295                 300
   gcc tcg cac ttc tcc aag tgt tat cgc gaa ctc tac aac cgc tcg ccg     1259
   Ala Ser His Phe Ser Lys Cys Tyr Arg Glu Leu Tyr Asn Arg Ser Pro
       305                 310                 315
   cag cag gag cgc gcc gac cgc aag ctg acg ctg cag atg gcg cga         1304
   Gln Gln Glu Arg Ala Asp Arg Lys Leu Thr Leu Gln Met Ala Arg
   320                 325                 330
   taagcggcag atcagatgga tcagacaagg cggagcttct ccgccctttt tcgttggtga   1364
   cactttccgc ttgtgccgtc ctggcgcttg cacgcgccgc tgttttggat tgaatggtcg   1424
   gcgtttcagg agagc                                                    1439
<210> 2
   <211> 334
   <212> PRT
   <213> Pseudomonas denitrificans
<400> 2
   Met Asn Lys Pro Leu Thr Lys Lys Arg Ser Leu Val Phe Phe Leu Val
   1 5 10 15
   Pro Asn Phe Ser Met Leu Pro Phe Ser Ala Ala Ile Glu Thr Leu Arg
   20 25 30
   Ile Ala Asn Arg Met Leu Gly Tyr Glu Ala Tyr Ser Trp Arg Leu Ala
   35 40 45
   Ser Ser Asp Gly Glu Lys Val Leu Ser Ser Ser Gly Ile Ala Leu Glu
   50 55 60
   Val Asn Ser Ser Leu Ala Asp Glu Arg Lys Phe Leu Gly Gly Glu Asn
   65 70 75 80
   Arg Pro Ser Met Val Leu Val Cys Ser Gly Ile Tyr Val Glu Asp Phe
   85 90 95
   Asn Asn Lys Ser Val Asn Ala Trp Leu Arg Glu Val Tyr Asn Arg Gly
   100 105 110
   Val Ala Val Gly Ser Leu Cys Thr Gly Ala His Val Leu Ala Ser Ala
   115 120 125
   Gly Leu Leu Thr Gly Lys Arg Cys Ala Ile His Trp Glu Asn Leu Pro
   130 135 140
   Gly Phe Ser Glu Ser Phe Pro Gln Val Asp Val Tyr Ala Asp Leu Tyr
   145 150 155 160
   Glu Ile Asp Ser Asn Ile Tyr Thr Cys Ala Gly Gly Thr Ala Ser Leu
   165 170 175
   Asp Met Met Leu Asn Leu Ile Asp Gln Asp Phe Gly Glu Ser Leu Val
   180 185 190
   Asn Arg Val Cys Glu Gln Ala Leu Thr Asp Arg Val Arg Gly Pro His
   195 200 205
   Asp Arg Gln Arg Leu Pro Leu Arg Ala Arg Leu Gly Val Gln Asn Ala
   210 215 220
   Lys Val Leu Ser Ile Ile Glu Leu Met Glu Ala Asn Leu Ala Glu Pro
   225 230 235 240
   Leu Ser Leu Leu Glu Ile Ala Glu Gly Ala Asp Leu Ser Arg Arg Gln
   245 250 255
   Ile Glu Arg Leu Phe Arg Gln Glu Met Gly Arg Ser Pro Ala Arg Tyr
   260 265 270
   Tyr Leu Glu Ile Arg Leu Asp Arg Ala Arg His Leu Leu Ile Gln Ser
   275 280 285
   Ser Met Pro Val Val Glu Val Ala Val Ala Cys Gly Phe Val Ser Ala
   290 295 300
   Ser His Phe Ser Lys Cys Tyr Arg Glu Leu Tyr Asn Arg Ser Pro Gln
   305 310 315 320
   Gln Glu Arg Ala Asp Arg Lys Leu Thr Leu Gln Met Ala Arg
   325 330
<210> 3
   <211> 20
   <212> DNA
   <213> Artificial Sequence
<220>
   <223> Primer N-GUS
<400> 3
   ccaggaatag gcctcgtagc 20
<210> 4
   <211> 31
   <212> DNA
   <213> Artificial sequence
<220>
   <223> Primer C-GUS
<400> 4
   cgcagagctc gagccaccga ggctgtagcc g 31
<210> 5
   <211> 32
   <212> DNA
   <213> Artificial sequence
<220>
   <223> oligonucleotide 1 for cassette
<400> 5
   ggatcctgac taactagcat ctggaggaca ca 32
<210> 6
   <211> 38
   <212> DNA
   <213> Artificial sequence
<220>
   <223> oligonucleotide 2 for cassette
<400> 6
   tatgtgtcct ccagatgcta gttagtcagg atcctgca 38
<210> 7
   <211> 25
   <212> DNA
   <213> Artificial sequence
<220>
   <223> Primer COBRNde
<400> 7
   ggcatatgaa caagccactg accaa 25
<210> 8
   <211> 34
   <212> DNA
   <213> Artificial sequence
<220>
   <223> Primer COBR135R
<400> 8
   gtcacccggg catatgttac gtcctgtaga aacc 34

## Description

The present invention relates to a transcriptional activator gene for genes involved in cobalamin biosynthesis. More precisely, it relates to a process for amplifying the production of cobalamins and, more specifically, of coenzyme B₁₂ by means of recombinant DNA techniques.

Vitamin B₁₂ is a member of molecules termed cobalamins and whose structure is presented, in particular, in WO91/11518. It has been used as agent for treating pernicious anemia, nervous diseases, methylmalonic aciduria, and so on, and feed additives for fowls and domestic animals. The industrial production of vitamin B₁₂ by chemical synthetic methods is difficult because of its complicated structure. Fermentation processes for industrial production use microorganisms selected from *Pseudomonas denitrificans*, *Propionibacterium shermanii* and *Propionibacterium freudenreichii.* The complicated biosynthetic pathway of vitamin B₁₂ has been studied well especially in *P. denitrificans, P. shermanii, Salmonella typhimuriuni,* and *Bacillus megaterium.* No less than 22 cob genes are involved in cobalamin biosynthesis (WO91/11518). However, the gene for an enzyme catalyzing reduction of cob(II)yrinic acid a,c-diamide has not been cloned and functions of polypeptides encoded by *cobE, cobW*, and *cobX* have not been identified.

Other genes involved in the biosynthesis of building blocks for cobalamins such as (R)-1-amino-2-propanol or 5,6-dimethylbenzimidazole (DMBI) were not identified in *P*. *denitrificans* although such genes were isolated from the photosynthetic bacterium *Rhodobacter capsulatus* (US 6,156,545). There are reports on a 189-kb segment of the chromosome of *R. capsulatus,* assigned as *cob* genes by analogy in this strain were also clustered and the blu genes involved in 1-amino-2-propanol and DMBI synthesis were found within the large cluster.

The vitamin B₁₂ productivity of *P. denitrificans* was gradually improved from 0.6 mg/l to 60 mg/l through repeated classical mutagenesis and random screening. The vitamin B₁₂ production process by *P. denitrificans* is characterized in that the fermentation is performed under aerobic condition from the first to the end contrary to the anaerobic fermentation or periodic fermentation from anaerobic to aerobic condition by *Propionibacterium.* In addition, it appears that glycine betaine (betaine) is an essential substance for overproduction of vitamin B₁₂ in *P. denitrificans.* Betaine is generally known as an osmoprotectant, but it was metabolized as N-source, C-source, and energy source in *P. denitrificans* as reported for the family *Rhizobiaceae.* Betaine is converted to dimethylglycine and methionine by transferring a methyl group to homocysteine by betaine homocysteine transmethylase. S-Adenosyl methionine synthesized from methionine is used as a methyl donor in the introduction of eight methyl groups to the corrinoid ring in vitamin B₁₂ synthesis. On the other hand, dimethylglycine synthesized from betaine is converted to glycine via sarcosine possibly by dimethylglycine dehydrogenase and sarcosine oxidase. Glycine is used for synthesizing 5-aminolevurinic acid (ALA), which is a common precursor for heme and vitamin B₁₂, with succinyl-CoA through C4-pathway (Shemin pathway). Thus, betaine is considered as a possible precursor for vitamin B₁₂ synthesis. But, the true role of betaine in cobalamin overproduction could be of a regulatory nature because the incorporation of radioactivity from [Me-¹⁴C]betaine to cobalamin was indirect. Moreover, blockage of betaine metabolism improved production of vitamin B₁₂. DNA fragments including genes involved in betaine metabolism were identified but the genes in the fragments were not identified. Therefore, they were no longer limited to the structural genes of enzymes involved in betaine metabolism such as betaine homocysteine transmethylase, dimethylglycine dehydrogenase, or sarcosine oxidase. A regulatory gene for the structural genes or genes involved in betaine uptake is also one of candidates.

The present invention provides a gene designated *cobR,* which encodes a transcriptional activator, designated CobR for genes involved in vitamin B₁₂ biosynthesis in *P*. *denitrificans* and the use of the gene or polypeptide for improving vitamin B₁₂ production or for screening of genes involved in vitamin B₁₂ synthesis.

The present invention additionally provides the use of CobR as a transcriptional activator for genes involved in betaine metabolism.

Although *cobR* was found to be an essential gene for betaine metabolism in *P. denitrificans,* restriction sites of *cobR* suggested that *cobR* was not included in the fragments as disclosed in US 5,691,163. Therefore, the present invention has made it possible to improve production of vitamin B₁₂ by appropriate expression of *cobR* without reducing betaine metabolism.

As described above, most genes involved in vitamin B₁₂ synthesis in *P. denitrificans* have been discovered and well characterized but some genes have remained to be discovered. To discover such genes we performed random mutagenesis and obtained several mutants that could not produce vitamin B₁₂. Among these mutants, there was a characteristic mutant of CEEX6, which neither produces vitamin B₁₂ nor utilizes betaine. By using this mutant, we found a novel gene *cobR* in *P. denitrificans,* which could make CEEX6 to produce vitamin B₁₂ and consume betaine simultaneously. CEEX6 has a point mutation in *cobR* and that *cobR* gene disruption lead to no production of vitamin B₁₂ and no consumption of betaine representing that *cobR* is an essential gene for vitamin B₁₂ synthesis and betaine consumption. These results suggested that CobR could be a transcriptional activator for genes involved in vitamin B₁₂ synthesis and betaine consumption although there were no examples of transcriptional activator for such genes in AraC/XylS family. Then, we demonstrated that CobR was a transcriptional activator for genes involved in vitamin B₁₂ synthesis by determining the promoter activity of *cobE* operon, which was a part of genes involved in vitamin B₁₂ synthesis, with the help of a reporter gene. CobR also interacts with upstream regions of *cobE, cobQ* and *cobF.* Appropriate expression of *cobR* increases vitamin B₁₂ production in *P. denitrificans.*

The present invention is directed to DNA sequences comprising a DNA sequence which encodes CobR, which is a transcriptional activator for genes involved in vitamin B₁₂ synthesis, as disclosed in the sequence listing, as well as their complementary strands, or those which include these sequences, DNA sequences which hybridize, under stringent conditions with such sequences or fragments thereof and DNA sequences, which because of the degeneration of the genetic code, do not hybridize under standard conditions with such sequences but which code for polypeptides having exactly the same amino acid sequence.

Protocols for identifying DNA sequences by means of hybridization are known to the person skilled in the art. The hybridization may take place under stringent conditions, that is to say only hybrids in which the probe and target sequence, i.e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps.

A 5x SSC buffer at a temperature of approx. 50-68°C, for example, can be employed for the hybridization reaction. Probes can also hybridize here with polynucleotides which are less than 70 % identical to the sequence of the probe. Such hybrids are less stable and are removed by washing under stringent conditions. This can be achieved, for example, by lowering the salt concentration to 2x SSC and subsequently 0.5x SSC at a temperature of approx. 50-68°C being established. It is optionally possible to lower the salt concentration to 0.1x SSC. Polynucleotide fragments, for example, at least 70% or at least 80% or at least 90% to 95% identical to the sequence of the probe employed can be isolated by increasing the hybridization temperature stepwise in steps of approx. 1-2°C.

"Stringent conditions" in the context of this invention mean that hybridization in a buffer, for example, consisting of 5x SSC, 0.1%(w/v) N-lauroylsarcosine, 0.02%(w/v) SDS, 1% blocking reagent (Roche Diagnostics, Cat. No. 1096 176) at 50°C overnight and two times of washing with 2 x SSC, 0.1%(w/v) SDS for 5 min at room temperature and following two times of washing with 0.1 x SSC, 0.1%(w/v) SDS for 15 min at 68°C in the washing step of hybridization.

The present invention also includes DNA sequences which are more than 90% identical to the DNA sequence according to SEQ ID NO:1 or a fragment thereof.

The present invention is also directed to functional derivatives of the polypeptides of the present invention. Such functional derivatives are defined on the basis of the amino acid sequences of the present invention by addition, insertion, deletion and/or substitution of one or more amino acid residues of such sequences wherein such derivatives still have the activity of the transcriptional activator for genes involved in vitamin B₁₂ synthesis. Such functional derivatives can be made either by chemical peptide synthesis known in the art or by recombinant means on the basis of the DNA sequence as disclosed herein by methods known in the state of the art. Amino acid exchanges in proteins and peptides which do not generally alter the activity of such molecules are known in the state of the art. The most commonly occuring exchanges are: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/gly as well as these in reverse.

Furthermore, polypeptides according to the invention include a polypeptide according to SEQ ID NO:2, in particular those with the biological activity of the transcriptional activator CobR for genes involved in cobalamin biosynthesis, and also those which are at least 95%, and particularly preferably more than 95% identical to the polypeptide according to SEQ ID NO: 2 and have the activity mentioned.

To express the *cobR* gene or generally speaking the CobR activity encoding DNA sequence efficiently, various promoters can be used; for example, the original promoter existing upstream of the *cobR* gene, promoters of antibiotic gene such as the kanamycin resistence gene of Tn5, the ampicillin resistence gene of pBR322, and of the β-galactosidase of *E*. *coli* (lac), the trp-, tac-, trc-promoter, promoters of lambda phage and any promoters which can be functional in the hosts consisting of microorganism including bacteria such as *E*. *coli, P. denitrificans, P. putida, Agrobacterium tumefaciens, A. radiobacter,* and *Sinorhizobium meliloti,* mammalian cells, and plant cells.

For the object above, other regulatory elements such as a Shine-Dalgarno (SD) sequence (for example, AGGAGG, and so on, including natural and synthetic sequence operable in the host cell) and a transcriptional terminator (inverted repeat structure including any natural and synthetic sequences operable in the host cell) which are operable in the host cell into which the coding sequence will be introduced and used with the above described promoter.

A wide variety of host/cloning vector combinations may be employed in cloning the double-stranded DNA. Cloning vector is generally a plasmid or phage which contains a replication origin, regulatory elements, a cloning site including a multiple cloning site and selection markers such as antibiotic resistance genes including resistance genes for ampicillin, tetracycline, chloramphenicol, kanamycin, streptomycin, gentamicin, spectinomycin etc.

Examples for a vector for the expression of the gene in *E*. *coli* are selected from any vectors usually used in *E*. *coli,* such as pBR322 or its derivatives including pUC18 and pBluescriptII, pACYC177 and pACYC184 and their derivatives, and a vector derived from a broad host range plasmid such as RK2 and RSF1010. A preferred vector for the expression of the object gene in *P. denitrificans, P. putida, A. tumefaciens, A. radiobacter,* or *S*. *meliloti* is selected from any vectors which can replicate in these microorganisms, as well as in a preferred cloning organism such as *E*. *coli.* The preferred vector is a broad host range vector such as a cosmid vector like pVK100 and its derivatives and RSF1010 and its derivatives, and a vector containing a replication origin functional in *P*. *denitrificans, P. putida, A. tumefaciens, A. radiobacter,* or *S. meliloti* and another origin functional in *E*. *coli.* Copy number and stability of the vector should be carefully considered for stable and efficient expression of the cloned gene and also for efficient cultivation of the host cell carrying the cloned gene. DNA sequences containing transposable element such as Tn5 can also be used as a vector to introduce the object gene into the preferred host, especially on a chromosome. DNA sequences containing any DNAs isolated from the preferred host together with the object gene are also useful to introduce the desired DNA sequence into the preferred host, especially on a chromosome. Such DNA sequences can be transferred to the preferred host by transformation, transduction, transconjugation or electroporation.

As a preferable microorganism, there may be mentioned bacteria such as *E. coli, P. denitrificans, P. putida, A. tumefaciens, A. radiobacter, S. meliloti,* and any Gram-negative bacteria which are capable of producing recombinant CobR. Mutants of said microorganism can also be used in the present invention.

The DNA sequence encoding CobR of the present invention may be ligated into a suitable vector containing a regulatory region such as a promoter and a ribosomal binding site and a transcriptional terminator operable in the host cell described above by well-known methods in the art to produce an expression vector.

To construct a host cell carrying an expression vector, various DNA transfer methods including transformation, transduction, conjugal mating, and electroporation can be used. The method for constructing a transformed host cell may be selected from the methods well known in the field of molecular biology. Usual transformation system can be used for *E. coli* and *Pseudomonas.* Transduction system can also be used for *E*. *coli.* Conjugal mating system can be widely used in Gram-positive and Gram-negative bacteria including *E. coli, P. putida, S. meliloti* and *P. denitrificans.*

Furthermore, the present invention relates to a process for screening of genes involved in vitamin B₁₂ synthesis by using the polypeptide according to SEQ ID NO: 2, in particular those with the transcriptional activator activity mentioned. Because a transcriptional activator activates transcription of a gene by interacting with DNA upstream the gene to be activated, a DNA fragment interacting with the transcriptional activator can be isolated from genomic DNA fragments using a polypeptide of transcriptional activator. It can be expected that genes involved in vitamin B₁₂ synthesis not only cob genes already isolated and identified are isolated by using affinity of polypeptide according to SEQ ID NO: 2 or its C-terminal part including DNA binding domain.

The following examples are offered by way of illustration and not by way of limitation.

### Strains and plasmids

*Pseudomonas denitrificans* PF1-48 is derived from strain MB580 (US 3,018,225) and it was deposited at DSMZ under the accession number DSM 15208 under the Budapest Treaty. Strain CEEX6 is a mutant, which does not consume betaine nor produce vitamin B₁₂, derived from PF1-48, and it was deposited at DSMZ under the accession number DSM 15207. Plasmid pGUS02, which was used in Example 3-(1), was constructed as follows. A promoter-less β-gluculonidase gene (*gus*) was prepared from pBI101 (CLONTECH Laboratories, Inc, CA) by PCR with the following primers; N-GUS: SEQ ID NO:3 (tagged with *Sma* I and *Nde* I sites) and C-GUS: SEQ ID NO:4 (tagged with *Xho* I and *Sac* I).
Then, the *Sma* I-*Sac* I fragment of *gus* was introduced between the *Sma* I and *Sac* I sites in pBluescriptII KS-(Stratagene, La Jolla, CA). At last, a cassette consisting of SD and three stop codons in three frames with *Pst I, Bam*H I, and *Nde* I sites (annealed product of SEQ ID NO: 5 and SEQ ID NO: 6) was introduced between *Pst* I and the constructed *Nde* I in the resulting plasmid.
Plasmid pETcobR, which was used in Example 3-(2), was constructed as follows. To make *Nde* I site at the initiation codon of *cobR,* PCR with primers COBRNde: SEQ ID NO: 7, and COBR135R: SEQ ID NO: 8 against pCRIIcobRB (pCRII::4.5 kb *Bam*HI) was performed. Then, the 0.1 kb *Nde* I*-Sph* I fragment from the PCR product was cloned into pUC18 between *Nde* I and *Sph* I sites and a clone that had the correct sequence was selected. Next, the 0.1 kb *Nde* I*-Sph* I fragment and a 1.1 kb *Sph* I*-Eco*R I fragment from pCRIIcobRB were cloned simultaneously into pUC18 between *Nde* I and *EcoR* I sites. At last, a 1.2 kb *Nde* I*-Eco*R I fragment was excised from the resulting plasmid and cloned into a modified pET11a (Stratagene) of which *Bam*HI site was converted to *Eco*R I site by using *EcoR* I linker: pGGAATTCC.

### Conjugation

Conjugations between *E*. *coli* ED8767 or DH5α and *P. denitrificans* strains were carried out by tri-parental conjugation method as follows. *E. coli* DH5 carrying pRK2013 having *tra* genes were used as a helper for conjugal transfer. *E. coli* strains were cultivated in LB with appropriate antibiotics at 37°C, and *P*. *denitrificans* strains were cultivated in LB at 30°C overnight. The portion of *E. coli* cultures were transferred to LB without antibiotics at 1% of inoculation size and cultivated for 6 hours. Cultures of *P*. *denitrificans,* donor *E. coli,* and helper *E*. *coli* were mixed at a ratio of 2 : 1 : 1 and the mixture was spotted onto nitrocellulose filter put on the LB agar plate. The plate was incubated at 30°C overnight for the mating. Then, transconjugants of *P. denitrificans* were selected from the cells grown on the filter by plating on LB agar with 100 µg/ml of streptomycin and 200 µg/ml of neomycin or 2.5 µg/ml of tetracycline.

### Gus assay

Cells from a portion (4 ml) of culture broth were harvested by centrifugation at 3,000 x g for 10 min at 4°C. The precipitate was washed with 3 ml of a buffer containing 25 mM Tris-HCl, pH 7.4 once and resuspended in the same buffer. After measurement of protein concentration of the cell suspension using BCA protein assay kit (Pierce, Rockford, ILL) with bovine serum albmin as a standard, the suspension was diluted with the buffer at the concentration of 1 mg protein/ml. The diluted suspension was dispensed into an Eppendorf tube by 50 µl and was frozen at -80°C once. The frozen sample was thawed at 4°C and 0.64 ml of Z buffer (60 mM Na₂HPO₄, 40 mM NaH₂PO₄, 10 mM KCl, 50 mM 2-mercaptoethanol-(2-ME), 1 mM MgSO₄·7aq, (add 2-ME just before use)) and 0.16 ml of Lysozyme solution (2.5 mg/ml Z buffer) were added to it. After mixing by vortex for 1 sec, the sample was incubated at 37°C for 5 min. Then, it was mixed for 1 sec. after addition of 8 µl of 10% Triton X-100 and was placed on ice. Next, it was pre-incubated at 30°C for 5 min and 400 µl of p-nitrophenyl-β-glucuronide (PNPG) solution (4 mg/ml Z buffer) was added. It was incubated at 30°C for 2 min. and 200 µl of 1M Na₂CO₃ was added quickly to stop the reaction. To remove cell debris, the reaction mixture was centrifuged at 12,000 rpm for 5 min and absorbance at 415 nm of the supernatant was measured. The molecular extinction coefficient of p-nitrophenol is assumed to be 14,000. One unit is defined as the amount of enzyme that produces one nmol of product per minute at 30°C.

### Assay of vitamin B₁₂ by HPLC

100 µl of 0.88 M acetate buffer (pH 3.5) with 0.11% KCN and 2.2% NaNO₂ were added to 1 ml of broth in a 2 ml Eppendorf tube with safe-lock. After mixing well with vortex, the mixture was autoclaved for 5 min with a tabletop steam sterilizer for cyanidation. The autoclaved sample was centrifuged for 5 min at 12,000 rpm and the supernatant was applied for HPLC assay. Ten µl of the sample was injected into Kanto Chemical Mightysil RP-18 GP (4.6 mm x 250 mm, 5 µm) column and eluted with 0.05 M KH₂PO₄: MeOH (75 : 25), pH 4.5 at flow rate of 0.8 ml/min. Vitamin B₁₂ eluted at about 10 min was detected by absorption at 361 nm.

### Assay of betaine by HPLC

After centrifugation of broth to remove cells, the supernatant was filtered with Ekicrodisc 25 (pore size 0.45 nm, Pall) and applied for HPLC assay. Ten µl of sample was injected into YMC-Pack PA (4.6 mm x 250 mm, 5 µm) column and eluted with Acetonitrile : H₂O (80 : 20) at flow rate of 2.0 ml/min. Betaine, sucrose, glucose, and fructose were detected by a Reflection Index detector R18021 (Tosoh Corporation, Tokyo, Japan).

### Media for producing vitamin B₁₂

The media used for vitamin B₁₂ synthesis are #688 for seed culture and CCM-1 for main production. One liter of #688 (pH 7.4 adjusted with 1 N NaOH) contains beat molasses (90g), (NH₄)₂HPO₄ (3 g), (NH₄)₂SO₄ (1.5 g), MgSO₄·7H₂O (1.5 g), and ZnSO₄·7H₂O (30 mg). One liter of CCM-1 (pH 7.4 adjusted with 1 N NaOH) contains sucrose (40 g), betaine (15 g), yeast extract Amberex 695AG (1g), citric acid (6 g), Na-glutamate (g), KH₂PO₄ (2 g), (NH₄)₂SO₄ (2 g), MgSO₄-7H₂O (4 g), FeSO₄.7H₂O (60 mg), ZnSO₄·7H₂O (20 mg), CaCl₂·2H₂O (0.5 g), MnSO₄-4-5H₂O (10 mg), CuSO₄·5H₂O (0.5 mg),
Co(NO₃)₂·6H₂O (250 mg) and 5,6-dimethylbenzimidazole (70 mg). The CCM-1X, which was used as a betaine-less medium in Example 3, is different from CCM-1 in content of betaine (0 g) and Na-glutamate (10 g).

### Example 1: Isolation of the cobR gene from P. denitrificans

### (1) Construction of a genomic library of P. denitrificans PF1-48

Chromosomal DNA prepared from *P. denitrificans* PFI-48 was partially digested with *Sal* I. and 15-35 kb fragments of the partial digests were isolated from preparative agarose gel electrophoresis (agarose: 0.7%); the gel piece containing the objective fragments was cut out and the fragments were electro-eluted into TAE buffer consisting of 40 mM Tris-acetate and 2 mM EDTA. In parallel, 5 µg of the cosmid vector pVK100 (ATCC37156) was completely digested with *Sal* I and the digest was treated with a shrimp alkaline phosphatase (United States Biochemical Corporation, Cleveland, USA) according to the supplier's recommendation. The dephosphorylated pVK100 (130 ng) was ligated with the 15-35 kb fragments (33 ng) by the DNA ligation kit ver.l (Takara Shuzo, Co. Ltd., Kyoto, Japan) at 26°C for 10 min. Then, the ligate was used for packaging by phage coat protein using λ *in vitro* packaging kit (Amersham Biosciences AB, Uppsala, Sweden) according to the supplier's protocol. The resulting phage particles were used for infection of *E*. *coli* ED8767, and about 400 kanamycin resistant colonies were obtained. These colonies were individually cultivated and the grown cells were stored at -80°C in 15% glycerol until use.

### (2) Screening of the genomic library for gene(s) complementing CEEX6

When CEEX6 is cultivated in CCM-1 medium, the culture broth does not become auburn because of no production of vitamin B₁₂. Using this characteristic of CEEX6, genomic library was screened for gene that makes CEEX6 produce auburn pigment. About 400 cosmid clones described above were individually transferred into CEEX6 from *E*. *coli* ED8767 by conjugation and the resulting transconjugants were cultivated in the 100 µl of CCM-1 in micro-titer plate with shaking at 30°C for 1 to 2 days. As a result, one clone CEEX6 carrying pS4F6 could make the medium auburn. Then, the vitamin B₁₂ productivity of the transconjugant in CCM-1 was evaluated in shaking flask cultivation. CEEX6 carrying pS4F6 recovered vitamin B₁₂ production and betaine consumption simultaneously.

### (3) Subcloning of a gene complementing CEEX6 in pS4F6

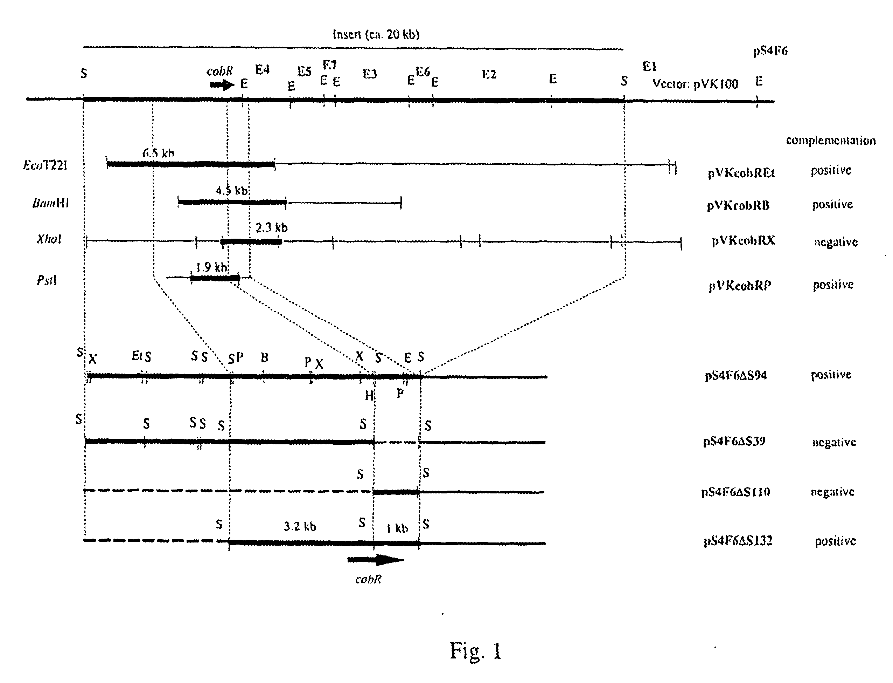

The localisation of the gene complementing CEEX6, i.e. *cobR,* in pS4F6 is shown in Fig. 1 where it is depicted with *EcoR* I sites. Subclones from pS4F6 are also shown with complementation ability of CEEX6 on pigment and vitamin B₁₂ production and betaine consumption. Each *Eco*R I fragment (E1-E6) does not complement CEEX6 in pigmentation ability (E7 was not tested). B: *Bam*H I, E: *EcoR* I, Et: *Eco*T22 1, H: *Hind* III, P*: Pst* I, S: *Sal* I, X: *Xho* I.

Several *Sal* I fragments from pS4F6 were deleted by partial digestion of pS4F6 with *Sal* I and re-ligation. 6 of *Eco*R I fragments (E1: 8 kb including 5.5 kb *Eco*R I*-Sal* I fragment from pVK100, E2: 5 kb, E3: 2.7 kb, E4: 1.8 kb, E5: 1.3 kb, E6: 1 kb) excised from pS4F6 were individually subcloned into pVK100. Existence of E7 (0.4 kb) was not recognized at this moment. About 5 kb fragment was estimated to remain at the vector which was not excised with *Eco*R I. Constructed deletion mutants with *Sal* I and pVK100 carrying each *Eco*R I fragment were transferred into CEEX6 and the resulting transconjugants were evaluated on pigment production in CCM-1.

As a result, each *EcoR* I fragment did not give CEEX6 pigmentation ability. On the other hand, one of deletion mutants with *Sal*I, pS4F6ΔS94, which had about 7 kb insert and only one *EcoR* I site in it, could make CEEX6 produce auburn pigment in the medium. However, pS4F6ΔS39, which was different from pS4F6ΔS94 in shortage of a 1 kb *Sal* I fragment, did not give the pigmentation ability to CEEX6. Then, further deletion mutants from pS4F6ΔS94 were constructed and analyzed. Plasmid pS4F6ΔS110, which had only the 1 kb *Sal* I fragment, did not show the pigmentation ability, while pS4F6ΔS132, which had the 1 kb *Sal* I fragment with the adjacent 3.2 kb one, showed the pigmentation ability. Therefore, the gene complementing CEEX6 should exist across the two *Sal* I fragments. Then, a 6.5 kb *Eco*T22 I fragment was cloned, which completely included these two *Sal* I fragments, from pS4F6 between two *Eco*T22 I sites in kanamycin resistance gene on pVK100 (pVKcobREt). In addition, 4.5 kb *Bam*H 1, 2.3 kb *Xho* I, and 1.9 kb *Pst* I fragments were prepared including the *Sal* 1 site between the two *Sal* I fragments using *Bam*H I*, Xho* I, or *Pst* I site in the 3.2 kb *Sal* I fragment. The 4.5 kb *BamH* I fragment was replaced with the 1.7 kb *Bgl* II fragment including cos site of pVK100 to construct pVKcobRB. The *Xho* I and *Pst* I fragments were introduced respectively at *Xho* I site and between two *Eco*T22 I sites in the kanamycin resistance gene on pVK100 to construct pVKcobRX and pVKcobRP. These clones were introduced into CEEX6 and assayed for vitamin B₁₂ productivity and betaine consumption activity. As shown in Table 1, all of the clones except for the clone carrying pVKcobRX and pVK100 recovered vitamin B₁₂ production and betaine consumption of CEEX6.

**Table 1**

| Strain | Vitamin B₁₂ production | Betaine consumption |
|---|---|---|
| CEEX6(pS4F6) | + | + |
| CEEX6(pVKcobREt) | + | + |
| CEEX6(pVKcobRB) | + | + |
| CEEX6(pVKcobRX) | - | - |
| CEEX6(pVKcobRP) | + | + |
| CEEX6(pVK100) | - | - + |
| PF1-48 | + | + |

### (4) Determination of the nucleotide sequence of the gene complementing CEEX6

In order to define the gene complementing CEEX6, the nucleotide sequence was determined with a focus on the 1.9 kb *Pst* 1 fragment, because it was the shortest fragment complementing CEEX6. We found one ORF of the gene which we designated *cobR* slightly across the *Pst* I site at the C-terminal. The ORF consisted of 1002 bp (SEQ ID NO:1: between nucleotide position 303 and 1304) encoding a polypeptide of 334 amino acids (SEQ ID NO:2). An *Xho* I site was found inside the ORF and it was consistent with the fact that pVKcobRX did not complement CEEX6. The gene product CobR could be a transcriptional activator belonging to AraC/XylS family because it had the helix-turn-helix motif found by Motifs progoram in GCG(Genetics Computer Group, University Research Park, WI, USA) conserved among the family near C-terminal; Hth_Arac_Family_1 motif (K,R,Q) (L,I,V,M,A) x2 (G,S,T,A,L,I,V) ~(F,Y,W,P,G,D,N) x2 (L,I,V,M,S,A) x{4,9} (L,I,V,M,F) x2 (L,I,V,M,S,T,A) (G,S,T,A,C,I,L) x3 (G,A,N,Q,R,F) (L,I,V,M,F,Y) x{4,5} (L,F,Y) x3 (F,Y,I,V,A) ~(F,Y,W,H,C,M) x3 (G,S,A,D,E,N,Q,K,R) x (N,S,T,A,P,K,L) (P,A,R,L) was conserved in CobR at 277th-319th residues as (R) (L) x{2} (A) ~(F,Y,W,P,G,D,N) x{2} (L) x{6} (V) x{2} (V) (A) x{3} (G) (F) x{5} (F) x{3} (Y) ~(F,Y,W,H,C,M) x{3} (N) x (S) (P). In the clone pVKcobRP, which carries the 1.9 kb *Pst* I fragment between two *Eco*T22I sites in pVK100, 4 amino acids at C-terminal of CobR (QMAR) would be replaced with 6 amino acids derived from the vector sequence (HHQEYG). This modified CobR could be functional to complement CEEX6. Homology search for *cobR* performed with Blast (NCBI, Bethesda, Md. USA) showed that it was homologous to a gene SMc04169 in the genome sequence of *Sinorhizobium meliloti* 1021 (Accession No. AL591688). Identity of *cobR* to SMc04169 was 86.6% at nucleotide level and 93.7% at amino acid level. The function of the gene product of SMc04169 was estimated as a transcriptional activator but it is hard to imagine that it can be an activator for genes involved in vitamin B₁₂ synthesis.

### Example 2: Confirmation of the relationship between the cobR mutation and deficiency in vitamin B₁₂ production and betaine consumption

### (1) Determination of a point mutation in the cobR from CEEX6

Introduction of *cobR* gene certainly complemented CEEX6, but it does not exactly mean CEEX6 has mutation in *cobR.* In order to confirm that the *cobR* mutation causes no consumption of betaine and no production of vitamin B₁₂, the *cobR* gene from CEEX6 was cloned and its nucleotide sequence determined as follows. A 2.1 kb fragment including the whole ORF of *cobR* was amplified from chromosome of strain PF1-48 by PCR, and it was used as a probe after labeling with DIG. Because we found that the 4.5 kb *Bam*H I fragment also existed in CEEX6 by Southern blotting analysis, the fragment was cloned by using colony hybridization method with the probe described above. Then, we confirmed that introduction of this 4.5 kb *Bam*H I fragment with pVK100 did not complement CEEX6. When a 2.1 kb *Bam*H I-*Hind* III fragment, which includes an N-terminal half of CobR, was replaced with a corresponding fragment from the *Bam*H I fragment from PF1-48, the resulting fragment also did not show the complementing activity. Therefore, we predicted that a mutation existed within the residual 2.4 kb *Hind* III-*Bam*H I fragment encoding a C-terminal half of CobR. In fact, we found a point mutation in the *cobR* gene of CEEX6 in the C-terminal region within the conserved region among AraC/XylS family transcriptional activator; Cys299(TGC) was changed to Arg299(CGC) in the *cob*R gene from CEEX6.

### (2) Random Tn mutagenesis against the 6.5 kb EcoT22I fragment including cobR

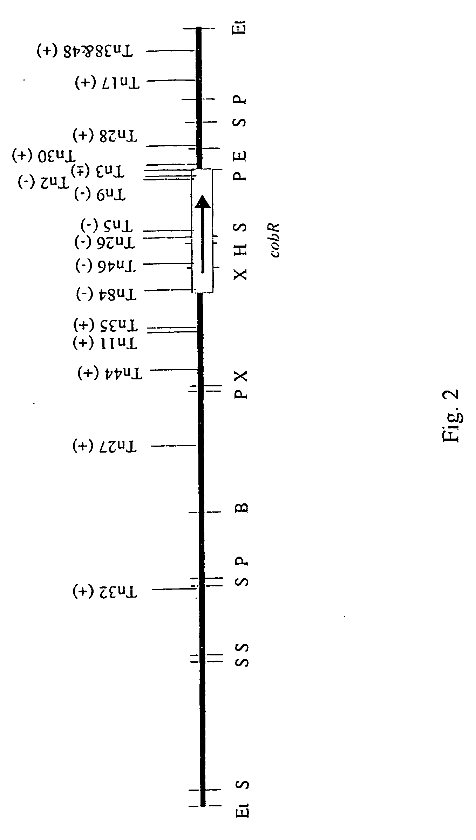

Using an *in vitro* Tn mutagenesis kit GSP-1 (New England BioLabs, Inc., Beverly, MA), pCRII vector (Invitrogen Corporation, Carlsbad, CA) carrying the 6.5 kb *Eco*T22 I fragment was mutated with pGPS2.1(CAT gene in the Tn). From the mutated sample, 8 kb *Eco*T22 I fragments including Tn were excised and introduced into pVK100 to transform *E.coli* DH5α. As a result, we obtained 128 Cm resistant transformants carrying various mutated fragments. Then, these plasmids were individually transferred into CEEX6 for testing complementation ability of pigmentation. Thirteen of 128 transconjugants showed no complementation ability. As summarized in Fig. 2, all of 6 negative clones carrying pVKTn2, 5, 9, 26, 46 or 84 analyzed out of 13 had a Tn in the ORF of *cobR.* On the other hand, Tn insertion surrounding *cobR* ORF such as Tn30 (81 bp downstream), Tn28 (about 300 bp downstream of *cobR* within a probable next ORF), or Tn35 (about 340 bp upstream) did not lose complementation ability. Plasmid pVKTn3, which exceptionally showed weak complementation ability, was inserted just at the *Pst* I site. Although substitution of 4 amino acid residues at C-terminal with 6 residues was possible in pVKcobRP, substitution of two (AR) with other 8 residues (WADNKVLN) was not acceptable.

Figure 2 shows random mutagenesis against 6.5 kb *Eco*T22 I fragment: Tn insertion sites investigated were mapped on the 6.5 kb *Eco*T22 I fragment. The ORF of *cobR* was shown as open box. Complementation ability of each fragment with Tn on pVK100 against CEEX6 were expressed as plus or minus in parenthesis with mapped Tn insertion site. B: *BamH* I, E: *EcoR* I, Et: *Eco*T22 I, H: *Hind* III, P: *Pst* I*,* S: *Sal* I, X: *Xho* I*.*

### (3) Construction of a cobR-null mutant

As described above, several fragments carrying Tn in *cobR* were obtained. Plasmid pVKTn26, which had a Tn insertion at the center of *cobR* between *Hind* III and *Sal* I (Fig. 2) was used for null mutant construction. The 1.9 kb *Pst* I fragment with Tn (3.3 kb in total) from pVKTn26 was inserted at the *Pst* I site of a suicide vector pSUP202ΔE, which was constructed by filling in the cohesive end of *EcoR* I site in pSUP202 to disrupt CAT gene on the plasmid. Then, using *Sal* I sites the CAT gene inside Tn together with a next *Sal* I fragment was replaced with gentamicin (Gm) resistance gene in the suicide plasmid. The resulting plasmid was introduced into PF1-48 and a Gm resistant cobr-null mutant Tn26 was obtained. Strain Tn26 did not produce vitamin B₁₂ nor consume betaine in CCM-1 medium at all as well as CEEX6. It means that *cobR* is one of essential genes for vitamin B₁₂ synthesis in *P. denitrificans.*

### Example 3: Demonstration of CobR involvement in activation of cob genes

Analyses of nucleotide sequence and deduced amino acid sequence suggested that *cobR* gene product CobR could be a transcriptional activator belonging to AraC family. Because *cobR* deficient mutants could not consume betaine nor produce vitamin B₁₂, we predicted that genes involved in betaine metabolism and vitamin B₁₂ synthesis were activated by CobR. In addition, betaine may be an effector molecule for CobR.

### (1) Estimation of cob gene expression level with or without CobR and betaine

We applied a promoter probe vector, pGus02, which was constructed using gus as a reporter gene, to estimate expression level of *cob* genes. We picked up *cobEABCD* operon as a representative of *cob* genes because *cobA* in the operon encodes the enzyme catalyzing the first step reaction in the vitamin B₁₂ synthetic pathway. In addition, we investigated expression level *of cobR* itself to know whether *cobR* was autoregulated or not. As a fragment including promoter for *cobEABCD* operon, a 0.7 kb *Hin*d III fragment covering 0.7 kb *Bgl* II-*Hin*d III fragment corresponding to 59th-792nd nucleotide in the sequence of *cobEABCD* genes resistered by Crouzet *et al.* in GenBank (Accession No. M59236), which has about 500 bp of 5'-noncoding region and about 200 bp of coding region of *cobE,* was inserted at *Hind* III site of pGus02. On the other hand, a 1.1 kb *Xho* I fragment, which has about 0.9 kb of 5'-noncoding region and 0.2 kb of coding region of *cobR,* was used as a fragment probably including a cob*R* promoter. The 1.1 kb *Xho* I fragment was inserted between two *Sal* I sites of pGus02a, which was constructed by insertion of multiple cloning sites from pUC19 between *Hin*d III and *BamH* I sites of pGus02. Then, units of *pcobE-gus* and *pcobR-gus* were respectively excised as 2.6 kb and 3 kb *Xho* I fragments from the vector and introduced at *Xho* I site of pVK100 in the reverse direction to kanamycin resistance gene on the plasmid to construct pVKpcobEgus and pVKpcobRgus, respectively. Then, the constructed plasmids were introduced into PF1-48 and CEEX6, and resulting transconjugants were cultivated in CCM-1 (betaine including medium) and CCM-1X (betaine-less but glutamate amplified from 1 to 10 g/l), in which B₁₂ was not produced though cells grew well. After two days cultivation, cells were collected and applied for gus assay. As shown in Table 2, we found that *cobE* promoter was repressed when cells were grown in the betaine-less medium or in the *cobR* deficient strain CEEX6. On the other hand, *cobR* promoter was not so repressed even in the betaine-less medium, although the activity in the *cobR* wild strain PF1-48 in CCM-1 was the highest. These results suggest that CobR activated *cobE* promoter along with betaine because *cobE* promoter was repressed though *cobR* could be expressed in the betaine-less medium and that CobR further activated own *cobR* promoter with betaine to some extent.

**Table 2**

| Strain | Medium | Promotor activity [gus activity (µmol/min/mg)] |
|---|---|---|
| | | [pcobR activity] |
| CEEX6/pVKpcobRgus | CCM-1 (+betaine) | 461.7 |
| PF1-48/pVKcobRgus | CCM-1 (+betaine) | 639.7 |
| CEEX6/pVKpcobRgus | CCM-1X (-betaine) | 389.3 |
| PF1-48/pVKcobRgus | CCM-LX (-betaine) | 303.5 |
| | | [pcobE activity] |
| CEEX6/pVKpcobEgus | CCM-1 (+betaine) | 91.2 |
| PF1-48/pVKcobEgus | CCM-1 (+betaine) | 570.0 |
| CEEX6/pVKpcoERgus | CCM-1X (-betaine) | 61.7 |
| PF1-48/pVKcobEgus | CCM-1X (-betaine) | 63.3 |
| | | (background) |
| CEEX6/pVK100 | CCM-1 (+betaine) | 45.0 |
| PF1-48/pVK100 | CCM-1 (+betaine) | 18.8 |
| CEEX6/pVK100 | CCM-1X (-betaine) | 19.8 |
| PF1-48/pVK100 | CCM-1X (-betaine) | 15.0 |

### (2) Demonstration of CobR function by using gel shift assay (CobR expressed in E. coli)

As described in the previous section, assay of promoter activity suggested that CobR activated *cobE* promoter with betaine. To get more direct evidence for CobR involvement in activation of *cob* genes, we conducted a gel shift assay. Instead of purified CobR, we used a cell free extract (CFE) of *E*. *coli* BL21(DE3)/pETcobR expressing CobR by the pET system (Stratagene) together with CFE of *E*. *coli* carrying only a pET vector as a negative control. E. *coli* cells cultivated in 2 x 10 ml of LB supplemented with ampicillin overnight were transferred to 2 x 200 ml of the same medium and cultivated for 4 hours at 37°C with shaking at 180 rpm. Cells were harvested by centrifugation at 4,000 x g for 10 min and washed once with 50 mM Tris-HCl (pH 7.5), 5 mM MgCl₂. The washed cells were resuspended in the same buffer at a concentration of 0.2 g of wet cell/ml. Then, CFE from the cell suspension was prepared by passing through French pressure cell twice at 1500 kg/cm² and removing cell debris by centrifugation at 3,000 x g for 10 min. On the other hand, we prepared a 290 bp (18 to 307 from the initiation codon) fragment upstream of *cobE* (cobEF1R1) by using PCR and labeled it at the 3'-end with D1G-11-ddUTP by terminal transferase included in DIG Gel Shift Kit (Roche Diagnostics, Basel, Switzerland).
According to the protocol recommended by the supplier, the gel shift assay was performed with the labeled fragment and the CFE. One ng of labeled fragment was incubated with CFE including 1.2 mg of protein in 15 µl of 20 mM HEPES, pH 7.6, 1 mM EDTA, 10 mM (NH₄)₂SO₄, 1 mM DTT, 0.2%(w/v) Tween 20, 30 mM KCI, 50 ng/µl Poly[d(I-C)], 5 ng/µl Poly L-lysine for 15 min at room temperature for binding reaction. After addition of 5 µl of 0.25 x 60% TBE buffer, 40% glycerol, 10 µl of the sample mixture was applied onto polyacrylamide gel electrophoresis (6% acrylamide in 0.25 x TBE, running buffer: 0.25 x TBE, constant voltage at 80 V for 2 hours). DNA and DNA-protein complex were electro-blotted onto positively charged nylon membrane (Boehringer Manheim GmbH Cat. No. 1209 299) by Trans-Blot SD (Bio-Rad Laboratories, Hercules, CA) using 0.25 x TBE as a transfer buffer at 25V for 45 min. Transferred DNA molecules were fixed on the membrane by baking the membrane at 120°C for 15 min and were detected according to the protocol recommended by the supplier. As a result, we observed retardation of the labeled fragment when it was reacted with the CFE including CobR. In addition, existence of 100-fold excess amount of non-labeled cobEF1R1 inhibited the retardation. On the contrary, the CFE from the vector control did not cause shift of the DNA fragment. These results strongly suggest that CobR directly binds to the regulatory region of *cobEABCD* operon to activate the transcription of the operon.

### (3) Demonstration of CobR involvement in activation of cob genes (CobR expressed in P. denitrificans)

We found that the CFE of *E. coli* including CobR was applicable for gel shift assay. It means we could demonstrate that CobR directly bound to the regulatory region of the *cobEABCD* operon. Then, CFE of the wild *P. denitrificans* strain PF1-48 was applied for gel shift assay in comparison with CFEs of CEEX6 (*cobR* with a point mutation) and Tn26 (*cob*R-null mutant) as negative controls. These strains were cultivated in 200 ml of LB for 16 hours at 30°C with shaking at 220 rpm, and CFE was prepared as well as *E*. *coli* cells described in Example 3-(2). Besides *cobEABCD* operon, region upstream the other *cob* genes were examined additionally. We prepared 208 bp (from T in the initiation codon) fragment upstream of *cobF* (cobFF1R1) and 281 bp (10 to 290 from the initiation codon) fragment upstream of *cobQ* (cobQF1R1) by PCR based on the sequences registered in Entrez (Accession No. A30008 and M62866). These fragments were labeled with DIG-11-ddUTP by terminal transferase and applied for gel shift assay.
As a result, we found that fragments from upstream region of *cobE* and *cobQ* clearly shifted only by CFE of PF1-48. On the other hand, although only a small part of fragments from upstream region of *cobF* shifted, the shift occurred when 10 mM of betaine was added into the binding reaction.

### Example 4: Purification of a truncated CobR for preparation of anti-serum against CobR

Although it was difficult to express *cobR* efficiently in *E. coli* as well as other transcriptional activators belonging to the AraC/XylS family even by using strong promoters such as tac or T7 in PET system, we found that deletion of N-terminal side at *Hind* III site (pCRIIcobRBΔH) of a clone carrying the 4.5 kb *BamH* I fragment in pCRII (pCRIIcobRB) could express truncated CobR fused with N-terminal of LacZ (20 a.a.) coming from the multiple cloning site (CobRONHd: 20+186/334 (CobR)=206 a.a., 23.4 kDa) efficiently as an inclusion body.without IPTG induction. Then, CobRΔNHd was purified from the inclusion body by preparative SDS-PAGE after solubilization with 8M urea as follows. By cultivating in 1L of LB medium supplemented with kanamycin, we obtained 3.3 g of wet cells of the recombinant E. *coli.* The cells were suspended in 16.5 ml of buffer A consisting of 50 mM Tris/HCl, 5 mM MgCl₂ (pH 7.5) and the suspension was passed through French pressure cell at 1500 kg/cm² twice. Next, inclusion body with cell debris was recovered from the solution by centrifugation at 3,000 x g for 10 min. The precipitate was washed with buffer A once, then solubilized with 2.5 ml of buffer B consisting of 100 mM NaH₂PO₄, 10 mM Tris, 8M urea (pH 8.0) by gentle mixing for one hour. The solubilized proteins were recovered by centrifugation at 3,000 x g for 10 min. At last, 125 mg protein in 2.5 ml buffer B was obtained. Two mg of protein was applied for preparative SDS-PAGE (15% acrylamide). The major protein band of CobRΔNHd was cut out from the gel, and the gel slice was provided to prepare rabbit anti-CobR antiserum.

### Example 5: DNA binding ability of the truncated CobR

Transcriptional activators that belong to AraC/XylS family have two HTH motifs near C-terminal. Sequence conservation at the first HTH motif is low and the highly divergent each other whereas the second HTH motif does not have such variation. The conserved sequence at the second HTH motif is a determining factor in classification of transcriptional activator into AraC/XylS family. As a matter of course CobR has the motifs in the C-terminal so that CobRΔNHd, which has C-terminal half of CobR, kept the DNA binding motifs. For the *Bacillus thermoglucosidasium* Hrc repressor it is reported that addition of any species of DNA appear to be effective for renaturation, and DNA containing Hrc binding site (CIRCE; controlling inverted repeat of chaperon expression) was far more effective than other nonspecific DNA. Then, the solubilized CobRΔNHd with 8 M urea in Example 4 was refolded under existence of DNA, which has CobR binding region. As such a DNA, we prepared pCRIIcobApd, which carries a 2.3 kb *Bgl*II-*Eco*R V fragment including *cobEA* genes in pCRII vector. Eighty µg of solubilized CobRANHd, 5 µg of pCRHcobApd in 100 µl of 20 mM Tris-HCl (pH 7.5), 5 mM EDTA, 3M urea was dialyzed against 20 mM Tris-HCl (pH 7.5), 5 mM EDTA. The dialyzate was diluted with 50 mM Tris-HCl (pH 7.5), 5 mM MgCl₂ to 1% (8 µg protein/ml). Using 1 µl of the solution instead of CFE and the labeled cobQF1R1 prepared in Example 3-(3) in the binding reaction described in Example 3-(2) gel shift assay was performed. As a result, we observed retardation of the fragment by renaturated CobRΔNHd. This result suggests that the N-terminal-truncated CobR or CobRΔNHd also has binding activity against regulatory region of *cob* genes.

### Example 6: Vitamin B₁₂ production by cobR-amplified PF1-48

PF1-48 carrying pVKcobRB and PF1-48 carrying pVK100 were cultivated in seed culture medium #688 for 2 days at 30°C. The portion (1 ml) of seed culture broth was inoculated into 30 ml of CCM-1 medium in a 500 ml EMF. These main fermentations were performed at 30°C, 220 rpm on a rotary shaker for 4 days. At the second day, 5 ml of feed medium consisting of 450 g of sucrose, 120 g of betaine, 3 g of MgSO₄·7aq, and 0.105 g of 5,6-dimethylbenzimidazole/L were added and the cultivation was continued for further two days. After cyanization of coenzyme B₁₂, quantity of vitamin B₁₂ produced was measured by HPLC. As a result, the former strain produced 6% higher amount of vitamin B₁₂ than the latter did.

## Claims

1. A process for the biological production of cobalamin which comprises introducing an isolated DNA into an appropriate host organism, cultivating the host organism under the condition conductive to the production of cobalamin and recovering cobalamin from the culture, said isolated DNA comprising a nucleotide sequence that encodes CobR, which is a transcriptional activator for genes involved in vitamin B₁₂ synthesis, selected from the group consisting of:
(a) a DNA sequence identified by SEQ ID NO:1 or the complementary strand thereof;
(b) a DNA sequence which hybridizes under stringent conditions to the DNA sequence complementary to the DNA sequence defined in (a) or a fragment thereof, and encodes a polypeptide having the activity of the transcriptional activator CobR for genes involved in vitamin B₁₂ synthesis:
(c) a DNA sequence which codes for a polypeptide having the amino acid sequence encoded by the DNA sequence of (a) or (b);
(d) a DNA sequence which is identical to the extent of at least 80% to a DNA which codes for a polypeptide which comprises the amino acid sequence of SEQ ID NO:2;
(e) a DNA sequence which is identical to the extent of at least 90% to a DNA which codes for a polypeptide which comprises the amino acid sequence of SEQ ID NO:2;
(f) a DNA which codes for a polypeptide which comprises an amino acid sequence which is identical to the extent of at least 80% to the amino acid sequence of SEQ ID NO:2;
(g) a DNA which codes for a polypeptide which comprises an amino acid sequence which is identical to the extent of at least 95% to the amino acid sequence of SEQ ID NO:2.

2. The process of claim 1, wherein said host organism is *Pseudomonas denitrificans, Agrobacterium radiobacter,* or *Sinorhizobium meliloti.*

3. The process of claim 2, wherein said host organism is *Pseudomonas denitrificans* CEEX6 or *Pseudomonas denitrificans* PF1-48, both deposited with the DSMZ under the Budapest Treaty, having deposit numbers DSM15207 and DSM15208, respectively.

4. A process for discovering genes involved in vitamin B₁₂ biosynthesis by using binding activity of a polypeptide encoded by the isolated DNA of any one of (a) to (g) in claim 1 against vitamin B₁₂ biosynthesis genes.

5. An isolated DNA comprising a nucleotide sequence that encodes CobR, which is a transcriptional activator for genes involved in vitamin B₁₂ synthesis, selected from the group consisting of:
(a) a DNA sequence identified by SEQ ID NO:1 or the complementary strand thereof;
(b) a DNA sequence which is more than 90% identical to the DNA sequence according to SEQ ID NO:1;
(c) a DNA sequence which codes for a polypeptide having the amino acid sequence encoded by the DNA sequence of (a);
(d) a DNA sequence which is identical to the extent of at least 90% to a DNA which codes for a polypeptide which comprises the amino acid sequence of SEQ ID NO:2;
(e) a DNA which codes for a polypeptide which has an amino acid sequence which is identical to the extent of at least 95% to the amino acid sequence of SEQ ID NO:2.

6. A vector or plasmid comprising the isolated DNA of any one of (a) to (e) in claim 5.

7. A host organism transformed or transfected by the isolated DNA as claimed in any one of (a) to (e) in claim 5 or by the vector or plasmid as claimed in claim 6.

8. A polypeptide encoded by the isolated DNA as claimed in any one of (a) to (e) in claim 5.

9. A process for the production of the polypeptide as claimed in claim 8 having activity of transcriptional activator for genes involved in vitamin B₁₂ synthesis, which comprises culturing the host cell as claimed in claim 7 under the conditions conductive to the production of said polypeptide, wherein the host cell is selected from the group consisting of *Pseudomonas denitrificans, Agrobacterium radiobacter, Agrobacterium tumefaciens.*

## Patentansprüche

1. Verfahren zur biologischen Erzeugung von Cobalamin, das beinhaltet, dass eine isolierte DNA in einen geeigneten Wirtsorganismus eingeführt wird, der Wirtsorganismus unter Bedingungen gezüchtet wird, die zur Erzeugung von Cobalamin führen, und Cobalamin aus der Kultur gewonnen wird, wobei die isolierte DNA eine Nucleotidsequenz enthält, die CobR codiert, das ein Transkriptionsaktivator für Gene ist, die an der Vitamin-B₁₂-Synthese beteiligt sind, ausgewählt aus der Gruppe bestehend aus:
(a) einer DNA-Sequenz, die in SEQ ID Nr. 1 angegeben ist oder einem komplementären Strang davon;
(b) einer DNA-Sequenz, die unter stringenten Bedingungen mit der DNA-Sequenz, die komplementär ist zu der DNA-Sequenz, die in (a) definiert ist, oder einem Fragment davon hybridisiert, und ein Polypeptid codiert mit der Aktivität des Transkriptionsaktivators CobR für Gene, die an der Vitamin-B₁₂₋Synthese beteiligt sind;
(c) einer DNA-Sequenz, die ein Polypeptid codiert mit der Aminosäuresequenz, die von der DNA-Sequenz von (a) oder (b) codiert wird;
(d) einer DNA-Sequenz, die zu mindestens 80% mit einer DNA identisch ist, die ein Polypeptid codiert, das die Aminosäuresequenz von SEQ ID Nr. 2 enthält;
(e) einer DNA-Sequenz, die zu mindestens 90% identisch ist mit einer DNA, die ein Polypeptid codiert, das die Aminosäuresequenz von SEQ ID Nr. 2 enthält;
(f) einer DNA, die ein Polypeptid codiert, das eine Aminosäuresequenz enthält, die zu mindestens 80% identisch ist mit der Aminosäuresequenz von SEQ ID Nr. 2;
(g) einer DNA, die ein Polypeptid codiert, das eine Aminosäuresequenz enthält, die zu mindestens 95% identisch ist mit der Aminosäuresequenz von SEQ ID Nr. 2.

2. Verfahren nach Anspruch 1, wobei der Wirtsorganismus *Pseudomonas denitrificans, Agrobacterium radiobacter* oder *Sinorhizobium meliloti* ist.

3. Verfahren nach Anspruch 2, wobei der Wirtsorganismus *Pseudomonas denitrificans* CEEX6 oder *Pseudomonas denitrificans* PF1-48 ist, die beide bei der DSMZ und nach Budapester Vertrag mit den Hinterlegungsnummern DSM 15207 bzw. DSM 15208 hinterlegt wurden.

4. Verfahren zum Auffinden von Genen, die an der Vitamin-B₁₂-Biosynthese beteiligt sind, wobei die Bindungsaktivität eines Polypeptids, das durch die isolierte DNA gemäß einer der Optionen (a) bis (g) in Anspruch 1 codiert wird, gegenüber Vitamin-B₁₂-Biosynthesegenen verwendet wird.

5. Isolierte DNA, die eine Nucleotidsequenz enthält, die CobR codiert, das ein Transkriptionsaktivator für Gene ist, die an der B₁₂-Synthese beteiligt sind, ausgewählt aus der Gruppe bestehend aus:
(a) einer DNA-Sequenz, die in SEQ ID Nr. 1 gezeigt ist, oder dem komplementären Strang davon;
(b) einer DNA-Sequenz, die zu mehr als 90% identisch ist mit der DNA-Sequenz gemäß SEQ ID Nr. 1;
(c) einer DNA-Sequenz, die ein Polypeptid codiert mit der Aminosäuresequenz, die von der DNA-Sequenz von (a) codiert wird;
(d) einer DNA-Sequenz, die zu mindestens 90% identisch ist mit einer DNA, die ein Polypeptid codiert, das die Aminosäuresequenz von SEQ ID Nr. 2 enthält;
(e) einer DNA, die ein Polypeptid codiert, das eine Aminosäuresequenz aufweist, die zu mindestens 95% identisch ist mit der Aminosäuresequenz von SEQ ID Nr. 2.

6. Vektor oder Plasmid enthaltend die isolierte DNA nach einer der Optionen (a) bis (e) von Anspruch 5.

7. Wirtsorganismus, der mit der isolierten DNA, die in einer der Optionen (a) bis (e) von Anspruch 5 beansprucht wird, oder dem Vektor oder Plasmid von Anspruch 6 transformiert oder transfiziert ist.

8. Polypeptid, das von der isolierten DNA nach einer der Optionen (a) bis (e) von Anspruch 5 codiert wird.

9. Verfahren zur Herstellung des Polypeptids nach Anspruch 8 mit einer Aktivität als Transkriptionsaktivator für Gene, die an der Vitamin-B₁₂-Synthese beteiligt sind, das beinhaltet, dass die Wirtszelle gemäß Anspruch 7 unter Bedingungen gezüchtet wird, die zur Erzeugung des Polypeptids führen, wobei die Wirtszelle ausgewählt ist aus der Gruppe bestehend aus *Pseudomonas denitrificans, Agrobacterium radiobacter, Agrobacterium tumefaciens.*

## Revendications

1. Procédé pour la production biologique de cobalamine qui comprend l'introduction d'un ADN isolé dans un organisme hôte approprié, la culture de l'organisme hôte dans les conditions conduisant à la production de cobalamine et la récupération de la cobalamine de la culture, ledit ADN isolé comprenant une séquence nucléotidique qui code CobR, qui est un activateur de transcription pour les gènes impliqués dans la synthèse de la vitamine B₁₂, choisi dans le groupe consistant en :
(a) une séquence d'ADN identifiée par SEQ ID NO : 1 ou son brin complémentaire ;
(b) une séquence d'ADN qui s'hybride dans des conditions strictes à la séquence d'ADN complémentaire à la séquence d'ADN définie dans (a) ou un fragment de celle-ci, et code un polypeptide ayant l'activité de l'activateur de transcription CobR pour les gènes impliqués dans la synthèse de la vitamine B₁₂ ;
(c) une séquence d'ADN qui code un polypeptide ayant la séquence d'acides aminés codée par la séquence d'ADN de (a) ou (b) ;
(d) une séquence d'ADN qui est identique au moins à 80 % à un ADN qui code un polypeptide qui comprend la séquence d'acides aminés de SEQ ID NO : 2 ;
(e) une séquence d'ADN qui est identique au moins à 90 % à un ADN qui code un polypeptide qui comprend la séquence d'acides aminés de SEQ ID NO : 2 ;
(f) un ADN qui code un polypeptide qui comprend une séquence d'acides aminés qui est identique au moins à 80 % à la séquence d'acides aminés de SEQ ID NO : 2 ;
(g) un ADN qui code un polypeptide qui comprend une séquence d'acides aminés qui est identique au moins à 95 % à la séquence d'acides aminés de SEQ ID NO : 2.

2. Procédé selon la revendication 1, dans lequel ledit organisme hôte est *Pseudomonas denitrificans, Agrobacterium radiobacter,* ou *Sinorhizobium meliloti.*

3. Procédé selon la revendication 2, dans lequel ledit organisme hôte est *Pseudomonas denitrificans* CEEX6 ou *Pseudomonas denitrificans* PF1-48, tous deux déposés auprès de la DSMZ selon le traité de Budapest, ayant des numéros de dépôt DSM15207 et DSM15208, respectivement.

4. Procédé de découverte de gènes impliqués dans la biosynthèse de la vitamine B₁₂ par utilisation de l'activité de liaison d'un polypeptide codé par l'ADN isolé de l'un quelconque de (a) à (g) selon la revendication 1 contre des gènes de la biosynthèse de la vitamine B₁₂ .

5. ADN isolé comprenant une séquence nucléotidique qui code CobR, qui est un activateur de transcription pour les gènes impliqués dans la synthèse de la vitamine B₁₂, choisie dans le groupe consistant en :
(a) une séquence d'ADN identifiée par SEQ ID NO : 1 ou son brin complémentaire ;
(b) une séquence d'ADN qui est identique à plus de 90 % à la séquence d'ADN selon SEQ ID NO : 1 ;
(c) une séquence d'ADN qui code un polypeptide ayant la séquence d'acides aminés codée par la séquence d'ADN de (a) ;
(d) une séquence d'ADN qui est identique au moins à 90 % à un ADN qui code un polypeptide qui comprend la séquence d'acides aminés de SEQ ID NO : 2 ;
(e) un ADN qui code un polypeptide qui a une séquence d'acides aminés qui est identique au moins à 95 % à la séquence d'acides aminés de SEQ ID NO : 2.

6. Vecteur ou plasmide comprenant l'ADN isolé de l'une quelconque de (a) à (e) selon la revendication 5.

7. Organisme hôte transformé ou transfecté par l'ADN isolé selon l'une quelconque des revendications de (a) à (e) selon la revendication 5 ou par le vecteur ou plasmide selon la revendication 6.

8. Polypeptide codé par l'ADN isolé tel que revendiqué dans l'un quelconque de (a) à (e) selon la revendication 5.

9. Procédé pour la production du polypeptide selon la revendication 8, ayant une activité d'activateur de transcription pour des gènes impliqués dans la synthèse de la vitamine B₁₂, qui comprend la culture de la cellule hôte selon la revendication 7 dans les conditions conduisant à la production dudit polypeptide, dans lequel la cellule hôte est choisie dans le groupe consistant en *Pseudomonas denitrificans, Agrobacterium radiobacter, Agrobacterium tumefaciens.*
